# EUROPEAN PATENT SPECIFICATION

(11) **EP 2 900 188 B1**
(45) Date of publication and mention of the grant of the patent: **05.12.2018**
(21) Application number: 13841828.0
(22) Date of filing: 30.09.2013
(51) Int. Cl.: A61L 29/06, A61L 29/14, A61L 29/16, A61F 11/00, A61F 2/18

(54) **BIO-ABSORBABLE MEDICAMENT-ELUTING VENTILATION TUBE**
BIOABSORBIERBARER MEDIKAMENTENELUIERENDER ATEMTUBUS
TUBE DE VENTILATION À ÉLUTION DE MÉDICAMENT BIO-ABSORBABLE

(30) Priority: 30.09.2012 US 201261708038 P
(43) Date of publication of application: 05.08.2015
(73) Proprietor: National University of Singapore, Singapore 119077 (SG); Nanyang Technological University, Singapore 637722 (SG)
(72) Inventor: LIM, Hsueh Yee, Lynne, Singapore 119077 (SG); GAN, Chee Wee, Singapore 119077 (SG); CHOOI, Wai Hon, Singapore 119077 (SG); VENKATRAMAN, Subramanian, Singapore 639798 (SG); NG, Herr Cheun Anthony, Singapore 639798 (SG); WONG, Yee Shan, Singapore 639798 (SG)
(74) Representative: Grosse Schumacher Knauer von Hirschhausen
(86) International application number: PCT/SG2013/000425
(87) International publication number: WO 2014/051524

(56) References cited:
- WO-A1-2012/128720
- US-A- 4 650 488
- US-A1- 2006 018 948
- US-A1- 2006 142 736
- US-A1- 2008 294 255
- US-A1- 2012 191 030
- US-B1- 6 589 286

## Description

### Technical Field

The present disclosure relates to implantable bio-degradable medical devices. Specifically, there is provided a medicament-eluting implantable biodegradable ventilation tube (VT).

### Background

Otitis-media with effusion (OME) is a term for inflammation and accumulation of liquid in the middle ear cleft. OME is a common ear disease worldwide, especially in children. Up to 93% of children experience at least one episode of OME during their childhood, while 74% of them experience three or more episodes. OME is the most frequent reason for prescribing medicaments to children.

When medical and conservative treatments fail to cure recurrent OME, tympanostomy and ventilation tube insertion are the standard follow-on treatments. In fact, since the introduction of ventilation tube insertion by Armstrong in 1952, it has become the most common surgery performed in the United States with over 0.5 million children undergoing ventilation tube insertion at an annual cost of up to 5 billion dollars.

A variety of ventilation tubes have been designed in different dimensions and shapes with the goal of increasing patency and tube retention. Commonly used ventilation tubes are made of stainless steel, fluoroplastics, titanium, or silicone each of which is susceptible to biofilm formation. Biofilm formation greatly contributes to post-tympanostomy-ventilation tubes otorrhea, which occurs in up to 50% of ventilation tubes implanted ears increasing tube occlusion and early tube extrusion. Materials forming ventilation tubes are not the only contributors to overall patency and retention of the ventilation tubes in the ear. Other factors, such as surface smoothness, tube weight, surface charge density, hydrophobitcity-hydrophilicity, and immune response to the implant also contribute to the effectiveness of ventilation tubes.

Most ventilation tubes are non-biodegradable and rely on squamous epithelial accumulation at the tympanic membrane for extrusion. Currently available biodegradable ventilation tubes are made of poly(D,L-lactide-co-glycolide) (PLGA) poly(L-lactide), and poly-bis(ethylanate). However, these tubes disintegrate about 30 days after implantation and only about 25% of these tubes are functional 60 days following implantation. While some ventilation tubes remain for only a few months, others stay for years. The longer a ventilation tube is present in the ear canal, the greater the risk of tympanosclerosis, biofilm, granulation tissue formation and permanent tympanic membrane perforation. However, the ventilation tube must remain in the ear for a long enough period of time for infection to resolve and drainage to stop. Furthermore, in cases of complications or blocked tubes, non-biodegradable tubes may have to be surgically removed requiring the patient undergo general anesthesia.
US 2012/0191030 A1 discloses stent devices for adequate ventilation and drainage for normal middle ear function having a shell including pharmaceutically active constituents.
US 2008/0294255 A1 discloses the placement of a tube for keeping open the ostia leading to the paranasal sinuses which can be coated with a coating containing a time release formulation of a pharmaceutical aent.
US 2006/0142736 A1 discloses tubular shaped devices used as drains for draining fluids from antrums or other parts of the human or animal body. The drain can comprise a biodegradable polymer optionally loaded with a pharmaceutical components.

### Summary

The present invention provides a bio-absorbable ventilation tube including a bio-absorbable co-polymer comprising polylactic acid and poly-ε-caprolactone and ofloxacin blended in with the polymer of the bio-absorbable ventilation tube, wherein 80% of said medicament releases from said bio-absorbable polymer in 90 days. Further disclosed herein is a customized bio-absorbable ventilation tube including a bio-absorbable polymer and at least one medicament, wherein said bio-absorbable polymer and said medicament are selected to produce a specific degradation rate *in vivo.*
Further disclosed herein is a method of preparing a customized bio-absorbable ventilation tube including identifying an aural malady; identifying length of treatment for said aural malady; selecting a combination of a medicament and a bio-absorbable polymer based on said aural malady and said length of treatment for said aural malady; and injection molding said combination,wherein said combination produces a customized bio-absorbable ventilation tube having an estimated *in vitro* degradation rate approximating the length of treatment of said aural malady.

### Brief Description of Drawings

Embodiments of the disclosure are described herein with reference to the drawings in which:
**FIG. 1** depicts the parts forming the middle ear of a human;
**FIG. 2A** depicts a top down view of an embodiment of a bio-absorbable ventilation tube of the present disclosure, without an added medicament;
**FIG. 2B** depicts a longitudinal view of an embodiment the bio-absorbable ventilation tube of FIG. 2A;
**FIG. 2C** depicts a top down view of an embodiment of a bio-absorbable ventilation tube of the present disclosure, with an added medicament;
**FIG. 2D** depicts a longitudinal view of an embodiment the bio-absorbable ventilation tube of FIG. 2C;
**FIG. 3** depicts a graph of the rate of medicament released during degradation of an embodiment of the bio-absorbable ventilation tube of the present disclosure;
**FIG. 4A** depicts a sterile fluoroplastic ventilation tube;
**FIG. 4B** depicts a fluoroplastic ventilation tube following exposure to *Pseudomonas aeruginosa;*
**FIG. 4C** depicts an embodiment of a bio-absorbable ventilation tube without a medicament in a sterile environment;
**FIG. 4D** depicts an embodiment of a bio-absorbable ventilation tube without a medicament following exposure to *Pseudomonas aeruginosa;*
**FIG. 4E** depicts an embodiment of a bio-absorbable ventilation tube containing a medicament in a sterile environment;
**FIG. 4F** depicts an embodiment of a bio-absorbable ventilation tube containing a medicament following exposure to *Pseudomonas aeruginosa*
**FIG. 5A** depicts an otoscopic image of a fluoroplastic ventilation tube;
**FIG. 5B** depicts an otoscopic image of an embodiment of a bio-absorbable ventilation tube containing a medicament;
**FIG. 5C** depicts an otoscopic image of an embodiment of a bio-absorbable ventilation tube without a medicament;
**FIG. 5D** depicts an SEM of the tympanic membrane of a guinea pig implanted with a fluroplastic ventilation tube;
**FIG. 5E** depicts an SEM of the tympanic membrane of a guinea pig implanted with an embodiment of the bio-absorbable ventilation tube containing a medicament;
**FIG. 5F** depicts an SEM of the tympanic membrane of a guinea pig implanted with an embodiment of the bio-absorbable ventilation tube without a medicament;
**FIG. 6A** depicts an otoscopic examination of a fluoroplastic ventilation tube;
**FIG. 6B** depicts an otoscopic examination of an embodiment of a bio-absorbable ventilation tube containing a medicament;
**FIG. 6C** depicts an otoscopic examination of an embodiment of a bio-absorbable ventilation tube not containing a medicament;
**FIG. 6D** depicts the extracted fluoroplastic ventilation tube of **FIG. 6A****;**
**FIG. 6E** depicts the extracted bio-absorbable ventilation tube containing a medicament of **FIG. 6B****;**
**FIG. 6F** depicts the extracted bio-absorbable ventilation tube not containing a medicament of **FIG. 6C****;**
**FIG. 6G** depicts a stained tympanic membrane following removal of the fluoroplastic ventilation tube of **FIG. 6A****;**
**FIG. 6H** depicts a stained tympanic membrane following removal of the bio-absorbable ventilation tube containing a medicament, of **FIG. 6B****;**
**FIG. 6I** depicts a stained tympanic membrane following removal of the bio-absorbable ventilation tube not containing a medicament, of **FIG. 6C****;**
**FIG. 7A** depicts a medial aspect of a dissected tympanic membrane of a guinea pig after 18 weeks implantation with a fluoroplastic ventilation tube;
**FIG. 7B** depicts a medial aspect of a dissected tympanic membrane of a guinea pig after 18 weeks implantation with an embodiment of a bio-absorbable ventilation tube containing a medicament;
**FIG. 7C** depicts a medial aspect of a dissected tympanic membrane of a guinea pig after 18 weeks following implantation of an embodiment of a bio-absorbable ventilation tube not containing a medicament;
**FIG. 7D** is an SEM image of a fluoroplastic ventilation tube in the tympanic membrane of a guinea pig 18 weeks after implantation;
**FIG. 7E** is an SEM image of an embodiment of a bio-absorbable ventilation tube containing a medicament in the tympanic membrane of a guinea pig 18 weeks after implantation; and
**FIG. 7F** is an SEM image of an embodiment of a bio-absorbable ventilation tube containing a medicament in the tympanic membrane of a guinea pig 18 weeks after implantation.

### Detailed Description

In the following detailed description reference is made to the accompanying drawings, which form a part hereof. In the drawings, similar symbols typically identify similar components, unless context dictates otherwise.
The present disclosure includes a bio-absorbable ventilation tube and methods for using the same. In embodiments, the bio-absorbable ventilation tube can include a bio-absorbable polymer and a medicament. In embodiments, the bio-absorbable ventilation tube can be placed in the ear.

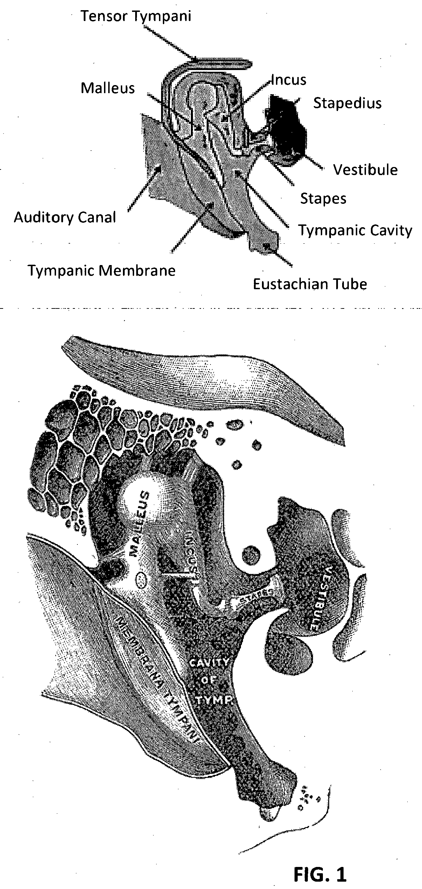

**FIG. 1** depicts the middle ear of a human. The middle ear includes the auditory canal, tympanic membrane, tensor tympani, malleus, incus, stapedius, vestibule, stapes, tympanic cavity, and Eustachian tube. In order to alleviate pressure in the middle or deliver medicaments to the inner ear, a myringotomy is often performed. In accordance with the present disclosure, the bio-absorbable ventilation tube of the disclosure can be inserted across the tympanic membrane during a myringotomy. In some embodiments, the bio-absorbable ventilation tube can be placed in the eardrum. In some embodiments, the bio-absorbable ventilation tube can be placed on the tympanic membrane.

The bio-absorbable ventilation tube can include one or more bio-absorbable polymers. In embodiments, the bio-absorbable polymers can be, for example, 3-hydroxypropionic acid (P(3-HP)), polylactic acid, poly L-lactide, poly D-lactide, poly DL lactide, polyglycolide, polycaprolactone, poly-ε-caprolactone, and the like, and copolymers and combinations of these. In embodiments, bio-absorbable ventilation tube includes a copolymer. In embodiments, the copolymer can include a polylactic acid-poly-ε-caprolactone copolymer.
In embodiments, the ratio of the polymers forming the copolymer can impact the bioabsorption rate of the bio-absorbable ventilation tube. In embodiments, as the presence and amount of lactic acid polymer in the bio-absorbable ventilation tube decreases, the rate of degradation can increase. In embodiments, the ratio of polylactic acid to another copolymer can be from 80:20 to 60:40. In embodiments, the ratio of polylactic acid to another copolymer can be 70:30. In embodiments, the ratio of polylactic acid to another copolymer can be adjusted and/or customized to accommodate the duration needed for the presence of the bio-absorbable ventilation tube in the ear.
The bio-absorbable ventilation tube disclosed herein can include a medicament. In embodiments, the medicament can be, for example, antibiotic, antifungal, anti-inflammatory agent, a drying agent, numbing agent, and the like, and combinations of these. In embodiments, the medicament can be a biologic for preventing attachment of bacteria or inflammatory cells, which can induce further inflammation, scaring, infection and the like at the site of insertion of the bio-absorbable ventilation tube, to the bio-absorbable ventilation tube. In embodiments, antibiotics can include any aurally acceptable antibiotic, for example, ofloxacin, ciprofloxacin, levofloxacin, sulfadimidine, tetracycline, polymyxin B, neomycin, bacitracin, erythromycin, azithromycin, gentamicin, framycetin, amoxicillin, phenethyl alcohol, cefuroxime, cefpodoxime, gramicidin, clarithromycin, and the like, and combinations of these. In embodiments, antifungal agents can include, for example, any antifungal that can contact the ear without causing damage, triamcinolone, nystatin, clotrimazole, chloroxylenol, and the like and combinations of these. In embodiments, anti-inflammatory agents can include, for example, prednisolone, triamcinolone, betamethasone, hydrocortisone, dexamethasone, and the like and combinations of these. In embodiments, drying agents can be used, such as acetic acid, aluminum acetate, sodium bicarbonate, and the like and combinations of these. In embodiments, the bio-absorbable ventilation tube can include a local or topical anesthetic. Topical anesthetics can include, for example, benzocaine, butamben, dibucaine, lidocaine, oxybuprocaine, pramoxine, proparacaine, proxymetacaine, tetracaine, and the like, and combinations of these. Biologics can include, for example, human serum albumin (HSA), collagen, and the like, and combinations of these. Any combination of medicaments can also be added to the bio-absorbable ventilation tube.

In embodiments, the type of medicament or medicaments added to the bio-absorbable ventilation tube disclosed herein can increase and/or decrease the rate of bio-absorption of the bio-absorbable ventilation tube. In embodiments, medicaments having a basic pH can increase degradation of the bio-absorbable ventilation tube and associated bio-absorption. In embodiments, medicaments having an acidic pH can increase degradation and bio-absorption of the bio-absorbable ventilation tube. In embodiments, medicament having a basic pH can increase bio-absorption by 25% to 50%. In embodiments, a medicament having an acidic pH can increase bio-absorption by 25% to 50%. In embodiments, the bio-absorbable ventilation tube can be customized to degrade a rate approximating the rate of treatment time for a specific aural malady.
In embodiments, the percentage of medicament added to the bio-absorbable ventilation tube can increase the bio-absorption rate of the bio-absorbable ventilation tube. In embodiments, the percentage of medicament added to the bio-absorbable ventilation tube can be from 0.1 wt % to 50 wt % as compared to the weight of the polymer(s). In embodiments, 2 wt% of a medicament can be added to a bio-absorbable ventilation tube. In embodiments, a bio-absorbable ventilation tube including 2 wt% medicament can have a bio-absorption rate of about 6 months. In embodiments, addition of 25 wt% of medicament to a bio-absorbable ventilation tube can increase the bio-absorption rate by 10% to 15% to 5 to 5.5 months.
In some embodiments, the bio-absorbable ventilation tube can be produced using injection molding. In embodiments, one or more polymers can be melted and injected into a mold to form the bio-absorbable ventilation tube. In some embodiments, one or more of the foregoing medicaments can be included in the polymer melt prior to injecting the melt into the mold. In embodiments, the medicament can be homogenized with the bio-absorbable polymer prior to injection molding.
In embodiments, the type of medicament added the bio-absorbable ventilation tube can be customized for a specific patient and/or treatment of a specific malady for a specific length of time. In accordance with the present disclosure, the customization of the bio-absorbable ventilation tube of the present disclosure affords the ability to alter the medicament depending upon, for example, what has or has not worked for a specific patient in the past, allergies a patient may have, tendency for scar tissue formation, tendency toward inflammation, and the like. In embodiments, the size and shape of the tube can also be customized depending on the intended usage and intended patient. Intended usage can include such as, for example, treatment of otitis media or other aural maladies, administration of trans-tympanic medicaments, administration of medicaments for other middle ear infections and or diseases. The intended patient may be any animal such as, for example, land mammals, like humans, dogs, cats, guinea pigs, gerbils, cows, goats, sheep, horses, and the like.
In embodiments, a specific combination of medicament and bio-absorbable polymer can have a certain degradation rate. In embodiments, the medicament and bio-absorbable polymer can be selected based on the length of treatment for a specific malady such that the degradation rate of the resulting bio-absorbable ventilation tube approximates the length of treatment for the specific malady.

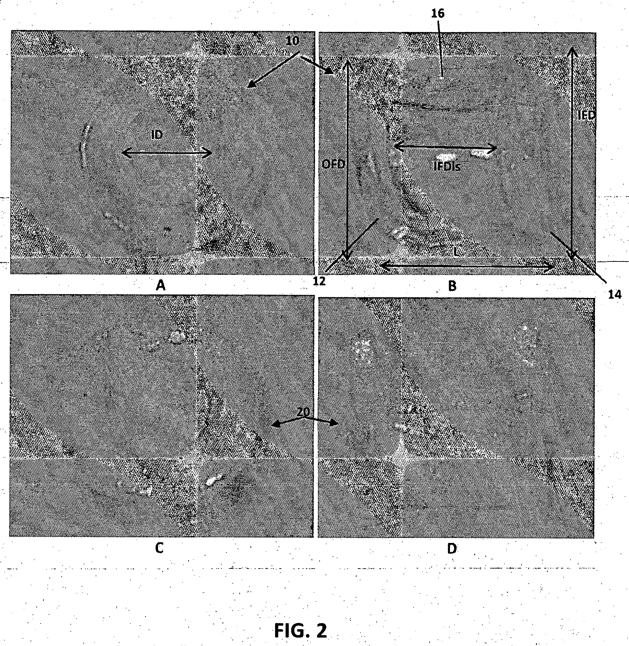

**FIG. 2A-B** depicts an embodiment of the bio-absorbable ventilation tube 10 of the present disclosure without an added medicament. FIG. **2C-D** depict an embodiment of the bio-absorbable ventilation tube 20 with an added medicament. As depicted in FIG. 2A-B, in embodiments, there can be two flanges, an outer flange 12 and an inner flange 14, separated by a shaft 16. In embodiments, bio-absorbable ventilation tube 10/20 can have a length (L). In embodiments, outer flange 12 can have an outer flange diameter (OFD) and the inner flange 14 can have an inner flange diameter (IFD). In embodiments, shaft 16 can have in inner diameter (ID) and an inter-flange diameter (IFDis).

In embodiments, the length of the bio-absorbable ventilation tube can be from 1.5 mm to 5 mm, in embodiments, the bio-absorbable ventilation tube can have a length of, for example, 1.6 mm, 2 mm, 2.1 mm, 3 mm, 4.7 mm, and any number in between these numbers. In embodiments, the outer flange and the inner flange can have a diameter of from 1.25 mm to 9.5 mm, for example, 1.27, 2.0, 2.1, 2.3, 2.4, 2.5, 2.6, 2.7, 2.8, 2.9, 3, 3.1, 3.5, 3.6, 4.3, 4.45, 5, 5.8, 9.5, and the like and any range between these numbers or within these numbers. In embodiments, the inner flange and the outer flange can have the same diameter, i.e., the IFD can equal the OFD. In embodiments, the IFD and the OFD can have different diameters. In embodiments, the IFD and OFD are each from 1.48 mm. In embodiments, the shaft can have an inter-flange diameter (IFDis) from about 0.5 mm to about 4.5 mm, for example, the IFDis can be 0.75, 0.76, 0.85, 0.9, 1, 1.1, 1.15, 1.3, 1.32, 1.5, 1.52, 1.55, 1.58, 1.75, 2.2, 2.5, and the like and any range between these numbers or within these numbers. In embodiments, the inner diameter of the shaft can be from 0.7 to 1.6, for example, 0.76, 0.97, 1.02, 1.14, 1.27, 1.52, and the like and any range between these numbers or within these numbers.
In some embodiments, the size of the bio-absorbable ventilation tube 10/20 can be customized for a specific purpose, for example the bio-absorbable ventilation tube 10/20 may be customized to have a length suitable for adult ear usage or child ear usage, or specific to the land animal in which the bio-absorbable ventilation tube is to be used.
In some embodiments, after injection molding the bio-absorbable ventilation tube, a coating can be applied to the lumen, shaft, one or both of the flanges, and combinations of these. The coating can include, for example, a medicament for immediate release, a lubricating coating for insertion, an antibody, human serum albumin (HSS), immunoglobulin, superparamagnetic iron oxide, selenium nanoparticles, and the like and combinations of these. In embodiments, the coating can reduce or prevent bacterial cell attachment to the bio-absorbable ventilation tube *in situ.* In embodiments, the coating can reduce or prevent inflammatory cell attachment to the bio-absorbable ventilation tube *in situ.* In embodiments, the coating can be customized to the suit the particular use of the bio-absorbable ventilation tube and/or the patient receiving the bio-absorbable ventilation tube. If a medicament is added to the coating, it may be the same as or different than the medicament added to the bio-absorbable ventilation tube prior to injection molding. In embodiments, a medicament intended to be used over an extended period of time can be added to the bio-absorbable ventilation tube prior to injection molding and an immediate acting medicament can be added to the bio-absorbable ventilation tube in the form of a coating.
In some embodiments, a medicament added to a bio-absorbable ventilation tube prior to injection molding can be released over a period of time. The period of time can be, for example, the time it takes for the bio-absorbable ventilation tube to be absorbed in situ. In embodiments, 5% to 10% of a medicament can be released from a bio-absorbable ventilation tube during the first 7 days following implantation. In embodiments, from 75% to 90% of the medicament added to the bio-absorbable ventilation tube prior to injection molding can be released by day 84 (week 12) following implantation.
In embodiments, in accordance with the present disclosure the bio-absorbable ventilation tubes (with or without addition of a medicament) do not induce serious inflammation, granulation formation, or otorrhea up to 18 weeks following implantation. In some embodiments, the bio-absorbable ventilation tube of the present disclosure can begin to degrade about 2.5 months following implantation. In some embodiments, the bio-absorbable ventilation tube of the present disclosure can continue to function for up to about 4.5 months after implantation. In some embodiments, the lumen patency of the bio-absorbable ventilation tube can be from about 2.5 months to about 6 months. In some embodiments, full degradation and bio-absorption of the bio-absorbable ventilation tube can be from about 4 months to about 12 months.

In embodiments, the bio-absorbable ventilation tube of the present disclosure can reduce complications due to prolonged retention of non-biodegradable ventilation tubes on the eardrum such as permanent ear drum perforation or granulation. In some embodiments, the injection molded biodegradable polymer used to form the bio-absorbable ventilation tube, can have a smooth tube surface. In embodiments, the smooth surface of the injection molded biodegradable polymer used to form the bio-absorbable ventilation tubes can inhibit bacterial adherence.

As stated above, in embodiments, the bio-absorbable polymer can include a medicament blended in with the polymer of the bio-absorbable ventilation tube. In embodiments, the medicament can inhibit bacterial growth or biofilm formation *in situ.* In embodiments, the inclusion of a medicament in the bio-absorbable polymer of the bio-absorbable ventilation tube can reduce the need for topical ear drops following tube insertion.

The present technology is further illustrated by the following examples, which should not be construed as in any way limiting.

### Examples

### Ventilation Tubes Used in further Examples

Poly I-lactide-co-ε-caprolactone copolymer: molar ratio of I-lactide to ε-caprotactone is 70:30; Mw = 202,000 g/mol (Purac, Schiedam, the Netherlands), was used in preparing bio-absorbable ventilation tubes using injection molding.

In preparing one of the tubes, 2 wt% of ofloxacin (Sigma-Aldrich Inc., St. Louis, MO) was dissolved with the co-polymer in dichloromethane and stirred until homogenous (over night). The mixture was then cast, dried and molded into a bio-absorbable ventilation tube.

The pure PLA-PCL bio-absorbable ventilation tube was transparent. The ofloxacin impregnated PLA-PCL appeared yellowish white.

### Example 1

### In vitro Degradation and Medicament Release

*In vitro* degradation was evaluated over 12 weeks.

Five tubes of each type (fluoroplastic, PLA-PCL containing ofloxacin, PLA-PCL without ofloxacin) were submerged in 2mL of water at a neutral pH in glass bottles and incubated at 37°C to simulate body temperature.

Size exclusion chromatography (SEC) was used to analyze the weighted-average molar mass, Mw, as an indicator of specimen degradation.

Specimens were dissolved in chloroform and filtered into SEC vials. Chloroform was used as a mobile phase.

Ofloxacin release was measured using Reverse Phase-High Performance Liquid Chromatography (RP-HPLC) measured at 294nm and a mobile phase of water/acetonitrile/acetic acid at a ratio of 4:1:0.025 with 0.25% ammonium acetate.

The molar mass decreased exponentially for PLA-PCL bio-absorbable ventilation tubes with or without ofloxacin over 12 weeks. A molar mass of 20,000 represents an average theoretical threshold value at which most polymers start to lose structure due to bulk degradation. Extrapolation (*R-Square* values close to 1.0) of the data predicted that bio-absorbable ventilation tube with and without ofloxacin would have lost integrity at approximately 3.5 and 4.2 months respectively.

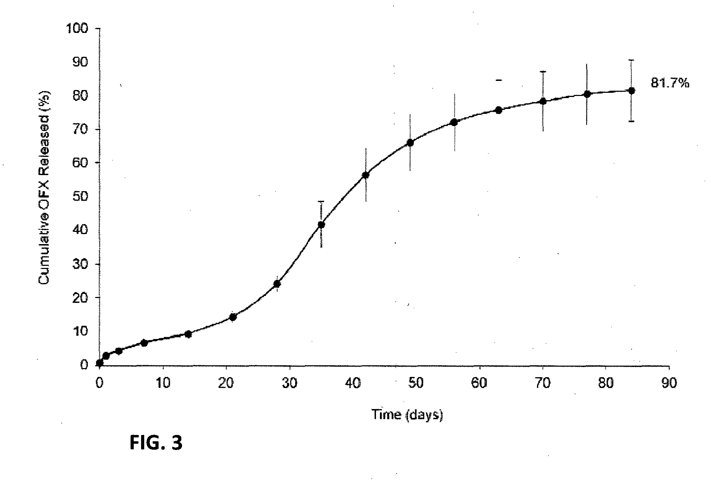

As depicted in FIG. 3, cumulative ofloxacin release over 12 weeks at 37°C in water showed an initial release of 6.6% in the first week, followed by a significant increase of medicament release rate between days 21-60. A more sustained release profile was seen thereafter with approximately 81.7% of the entrapped medicament released after 84 days.

### Example 2

### In vitro Bacterial Adherence Study

Two sets of three sterilized tubes (fluoroplastic, pure PLA-PCL, PLA-PCL with ofloxacin) were used. One set was kept as negative control (sterilized). Another positive control set was incubated in Trypton Soy Broth (TSB) containing 1.5 x 10⁸ CFU/ml *Pseudomonas aeruginosa* (measured by Optical Density at 600 nm).

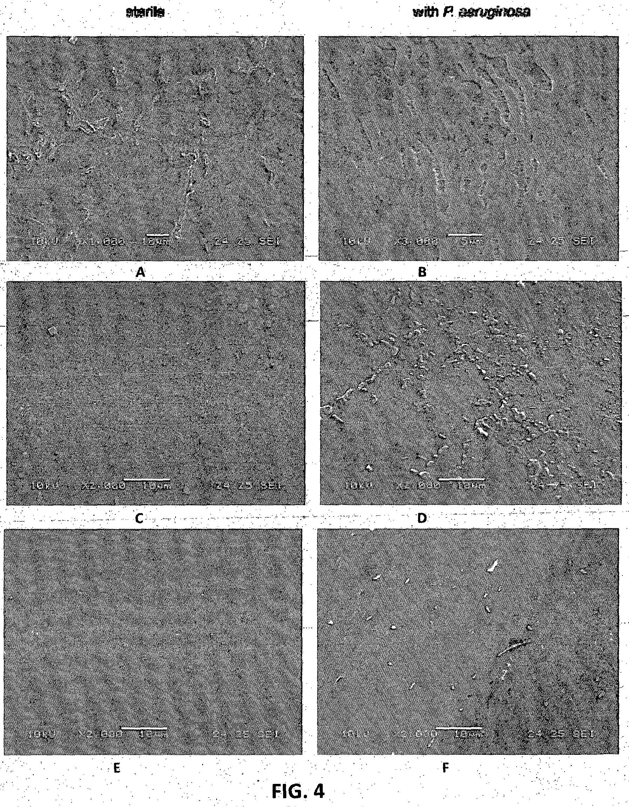

Mixtures were incubated at 37°C for 6 days. As depicted in the scanning electron microscopy (SEM) images of **FIG. 4****,** the surface of the control was much rougher (**FIG. 4A-B**) than that of the PLA-PCL bio-absorbable ventilation tube with ofloxacin (**FIG. 4E-F**) or without ofloxacin (**FIG. 4C-****D**). The surface of the PLA-PCL bio-absorbable ventilation tube was smooth with negligible grooves. Most of the crystal-like particles seen on the surface of the sterile PLA-PCL bio-absorbable ventilation tube were attributed to the crystalline salt from buffered solution during SEM preparation.

After 6 days of incubation with gram-negative *P*. *aeruginosa,* colonies of aggregated rod-shaped bacteria were found on the surface of the fluoroplastic tube. A higher magnification of the image showed clumps of bacteria connecting to each other with fibrous-like structures, mostly due to the initial stage of 3-dimensional biofilm formation characterized by blocks of bacterial conglomerations. The PLA-PCL tube without the ofloxacin showed less bacterial attachment as compared to the fluoroplastic tube, with bacteria only scattered individually on the surface. PLA-PCL tube with ofloxacin had even less bacteria attached on the surface than the PLA-PCL tube without the ofloxacin.

### Example 3

### Animal Study

Hartley guinea pigs (600-700g) were supplied by Laboratory Animal Centre, Singapore, Animals were acclimatized for 4-5 days before undergoing experimentation.

Conventional myringotomy using operating microscope (OPMI Pico, Carl Zeiss, Germany) was performed on guinea pigs under isofluorane anesthesia. A fluoroplastic tube was used as a positive control and PLA-PCL bio-absorbable ventilation tubes were place on the anterior side of the tympanic membrane in an aseptic manner.

Guinea pigs were randomly divided into two groups, PLA-PCL tube with 2 wt% ofloxacin (Group A) and PLA-PCL tube without medicament (Group B). Fluoroplastic tubes were inserted into one of the ears of each guinea pig for comparison (control). An endoscope (1.9mm Telescope 0°, Karl Storz, Germany) was used to perform gross examination of the implanted ear weekly.

At certain time points, some guinea pigs were sacrificed via CO₂ inhalation. Tympanic bullae were then dissected from the animals via post-auricular approach. Bullae were dissected to expose the medial aspect of the tympanic membrane for scanning electron microscopy (SEM) and histology. All explanted ventilation tubes were analyzed for degradation.

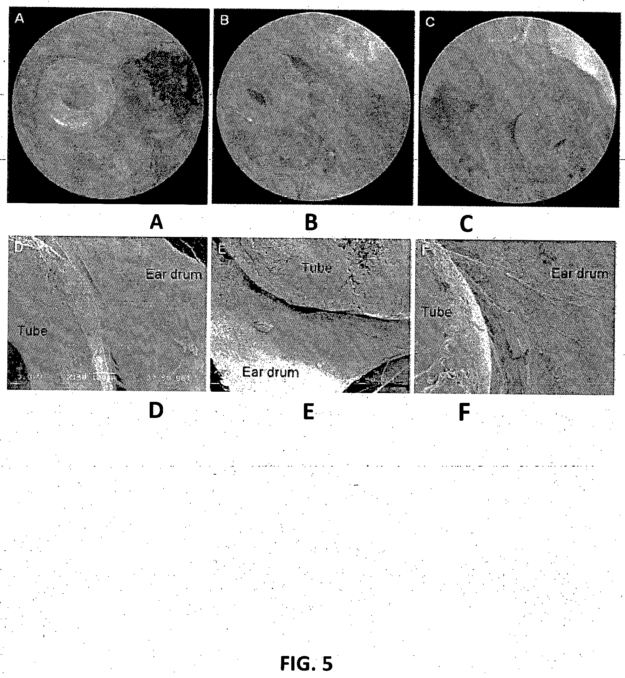

Otoscopic images (**FIG. 5A-C**) and SEM (**FIG. 5D-F**) of the dissected tympanic bullae at 30 days after myringotomy did not show any appreciable inflammation or redness of the membrane or granulation tissue formation. The tympanic membrane around the ventilation tube showed some scar tissue due to the healing of myringotomy incisions. The flanges of PLA-PCL bio-absorbable ventilation tube with or without ofloxacin had slight aggregates caused by dust particles.

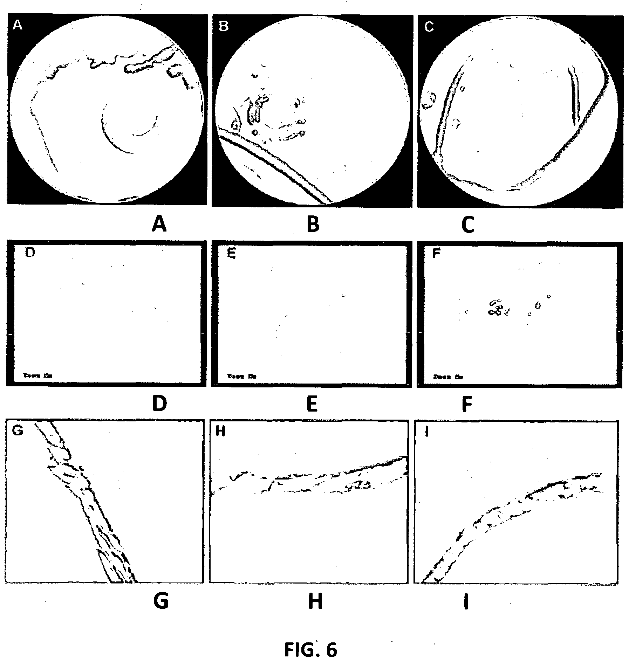

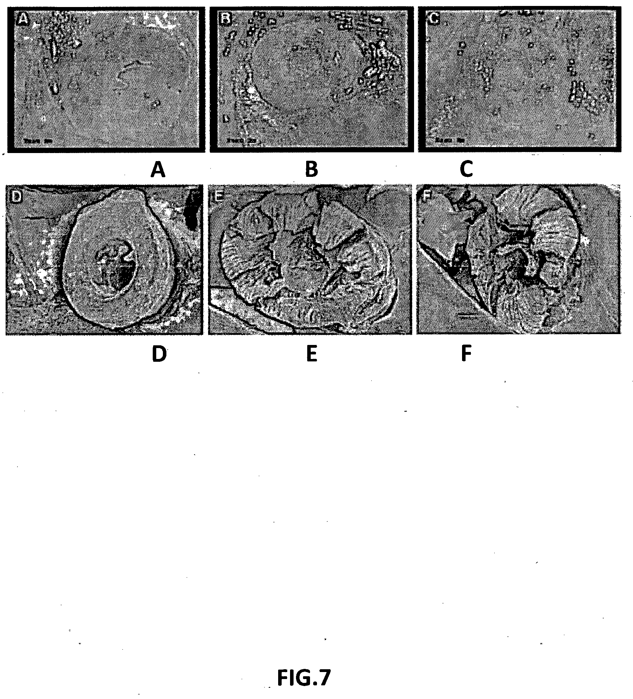

As shown in **FIG. 6A-C****,** no obvious inflammation or granulation tissue was present 18 weeks after implantation. The basic structural layers of the tympanic membrane were preserved. As shown in **FIG. 6D-F****,** the bio-absorbable ventilation tube containing the ofloxacin (**FIG. 6E**) had begun to crack and after 18 weeks while the bio-absorbable ventilation tube without the ofloxacin (**FIG. 6E**) was less degraded than the bio-absorbable ventilation tube containing the ofloxacin (**FIG. 6F**). The fluoroplastic ventilation tube did not exhibit degradation (**FIG. 6D**). Hematoxylin and eosin staining of the tympanic membrane close to the ventilation tube-tympanic membrane interface further confirmed that the basic structural layers of the tympanic membrane were preserved (see **FIG. 6G-I**). Gross tissue examination from dissected tympanic bulla revealed the same (see **FIG. 7**). The lumens remained clear with no evidence of clogging.

As depicted in **FIG. 7A-D****,** 18 weeks following implantation, the PLA-PCL bio-absorbable ventilation tube, with and without ofloxacin were still functional but as depicted in **FIG. 7E-F****,** the PLA-PCL bio-absorbable ventilation tube with and without ofloxacin had both begun to degrade 18 weeks after implantation. The fluoroplastic ventilation tube did not show signs of degradation (**FIG. 7D**).

## Claims

1. A bio-absorbable ventilation tube comprising:
a bio-absorbable co-polymer comprising polylactic acid and poly-ε-caprolactone; and
ofloxacin blended in with the polymer of the bio-absorbable ventilation tube,
wherein 80% of said medicament releases from said bio-absorbable polymer in 90 days.

2. The bio-absorbable ventilation tube of claim 1, wherein the bio-absorbable ventilation tube has an outer flange diameter of from 1.25 mm to 2.1 mm.

3. The bio-absorbable ventilation tube of claim 1, wherein the bio-absorbable ventilation tube has an inner diameter of from 0.7 mm to 1.14 mm.

4. The bio-absorbable ventilation tube of claim 1, wherein the medicament comprises from 0.1 wt. % to 50 wt. % of the bio-absorbable ventilation tube.

## Patentansprüche

1. Ein bioabsorbierbarer Belüftungsschlauch, umfassend:
ein bioabsorbierbares Co-Polymer, welches Polymilchsäure und Poly-ε-Caprolacton beinhaltet; und
Ofloxacin, das mit dem Polymer des bioabsorbierbaren Beatmungsschlauchs vermischt ist,
wobei etwa 80% des Medikaments in etwa 90 Tagen aus dem bioabsorbierbaren Polymer freigesetzt werden.

2. Der bioabsorbierbare Belüftungsschlauch nach Anspruch 1, wobei der bioabsorbierbare Belüftungsschlauch einen äußeren Flanschdurchmesser von etwa 1,25 mm bis etwa 2,1 mm aufweist.

3. Der bioabsorbierbare Belüftungsschlauch nach Anspruch 1, wobei der bioabsorbierbare Belüftungsschlauch einen Innendurchmesser von etwa 0,7 mm bis etwa 1,14 mm aufweist.

4. Der bioabsorbierbare Belüftungsschlauch nach Anspruch 1, worin das Medikament von etwa 0,1 Gew.-% bis etwa 50 Gew.-% des bioabsorbierbaren Belüftungsschlauches umfasst.

## Revendications

1. Tube de ventilation bio-absorbable comprenant :
un copolymère bio-absorbable comprenant de l'acide polylactique et du poly-ε-caprolactone ; et
du ofloxacine mélangé dans le polymère du tube de ventilation bio-absorbable,
ledit polymère bio-absorbable étant destiné à libérer 80% dudit médicament dans 90 jours.

2. Tube de ventilation bio-absorbable selon la revendication 1, ledit tube de ventilation bio-absorbable ayant un diamètre extérieur de la bride allant de 1,25 mm à 2,1 mm.

3. Tube de ventilation bio-absorbable selon la revendication 1, ledit tube de ventilation bio-absorbable ayant un diamètre intérieur allant de 0,7 mm à 1,14 mm.

4. Tube de ventilation bio-absorbable selon la revendication 1, ledit médicament constituant un pourcentage en poids du tube de ventilation bio-absorbable allant de 0,1 % à 50 %.
